# EUROPEAN PATENT SPECIFICATION

(11) **EP 2 809 236 B1**
(45) Date of publication and mention of the grant of the patent: **11.12.2019**
(21) Application number: 13776159.9
(22) Date of filing: 21.02.2013
(51) Int. Cl.: A61B 8/12, G01S 15/89, H01R 12/79, G01S 7/52, A61M 25/01, A61B 8/08

(54) **SYSTEM COMPRISING A MEDICAL DEVICE AND A PAIR OF WIRE HARNESSES**
SYSTEM MIT EINER MEDIZINISCHEN VORRICHTUNG UND EINEM KABELBAUMPAAR
SYSTÈME COMPRENANT UN DISPOSITIF MÉDICAL ET UNE PAIRE DE FAISCEAUX DE CÂBLES

(30) Priority: 09.04.2012 US 201261621814 P; 07.05.2012 US 201213465655
(43) Date of publication of application: 10.12.2014
(73) Proprietor: St. Jude Medical Atrial Fibrillation Division, Inc., St. Paul, MN 55117-9913 (US)
(72) Inventor: TEGG, Troy, T., Elk River, Minnesota 55330 (US)
(74) Representative: Kramer Barske Schmidtchen Patentanwälte PartG mbB
(86) International application number: PCT/US2013/026990
(87) International publication number: WO 2013/154684

(56) References cited:
- EP-B1- 0 431 206
- EP-B1- 0 431 206
- WO-A2-98/33428
- WO-A2-2009/149315
- GB-A- 2 315 020
- US-A- 4 924 092
- US-A- 5 931 577
- US-A1- 2008 312 536
- US-A1- 2008 312 536
- US-B1- 6 211 936
- US-B2- 7 901 358
- US-B2- 7 901 358
- Jeff Strole ET AL: "A Novel Flex Circuit Area-Array Interconnect System for a Catheter-Based Ultrasound Transducer", , 5 September 2002 (2002-09-05), XP055215283, Retrieved from the Internet: URL:https://www.microconnex.com/publicatio ns/imaps_2001.pdf [retrieved on 2015-09-22]

## Description

### BACKGROUND OF THE INVENTION

### a. Field of the Invention

This disclosure relates generally to the field of medical devices, including a medical device for introduction into a body, such as a catheter, and other maneuverable medical devices.

### b. Background Art

WO 2009/149315 A2 is directed to the area of transducers for ultrasound imaging systems including a catheter and an imaging core, wherein the imaging core includes a rotatable drive shaft, at least one transducer mounted to a distal end of the rotatable drive shaft and a twisted wire cable coupled to the at least one transducer. The twisted wire cable includes two wires twisted together along the cable and electrically coupled to respective contacts of the at least one transducer, and a conductive shield extending along the cable and within which a portion of the two wires are disposed.

GB 2 315 020 A relates to a balloon catheter including an ultrasonic transducer at its distal end.

US 7,901,358 B2 relates to a system for acquiring an ultrasound signal comprised of a signal processing unit adapted for acquiring a received ultrasound signal from an ultrasound transducer having a plurality of elements.

WO 98/33428 A2 relates to ultrasonic transducer assemblies of the type used with medical diagnostic imaging systems, wherein by providing a relatively inexpensive interconnection system between a transducer assembly and a receptacle, a low-cost transducer assembly can be provided that can be considered disposable for many applications.

US 2008/0312536 A1 is directed toward providing compact, portable ultrasound systems, which can generate images suitable for rendering three-dimensional images of organs.

EP 0431206 B1 relates to a grounding device, in particular to the use of commonly available electrical disconnect devices and tooling usually identical to those used to terminate the wires in a connector.

"A Novel Flex Circuit Area-Array Interconnect System for a Catheter-Based Ultrasound Transducer"; Jeff Strole, Scott Corbett, Warren Lee, Edward Light, Stephen Smith; presented at IMAPS 2002, Denver Colorado, September 5, 2002 (Best of Session Award) discloses a flex circuit area-array interconnect system for a catheter-based ultrasound transducer, wherein a first wire harness and a second wire harness are not each operatively connected to the same flexible circuit board for an electrically controlled object.

Catheters and sheaths having flexible tubular bodies with deflectable distal ends and control handles for controlling distal end deflection are commonly used in connection with many noninvasive medical procedures. For example, catheters having one or more ultrasound transducers along the distal ends of their bodies are used for intra-cardiac echocardiography studies. The distal end of the catheter body is typically positioned in a patient's heart and an ultrasound transducer may provide signal data which may be used to generate images to visualize cardiac structures and blood flow within the heart during intra-cardiac visualization, navigation, and mapping. Generally, an ultrasound transducer may comprise one piezoelectric element or a plurality of piezoelectric elements. Each piezoelectric element may have a relatively fine electrically conductive wire attached thereto and the wire may extend through the catheter body, ultimately to an electronic control unit (ECU). For example, the conductive wire may extend from the distal end to a proximal end of the catheter where the wire may be terminated with an electrical connector that can be configured to connect with a corresponding socket provided in an ECU. To organize a plurality of wires running throughout the catheter body, the wires may be positioned and attached on a flat mylar ribbon.

In an effort to obtain sharper images, ultrasound transducers having an increased number of elements may be utilized. However, an increased number of elements can also increase the number of associated or corresponding conductive wires extending through the catheter body. An increased number of conductive wires extending through the catheter body may decrease the flexibility and maneuverability of the catheter by increasing the stiffness associated with the wiring. Further, when wiring is provided on flat ribbons, the amount of flexibility may be inconsistent depending on the direction of the flex or bend of the body relative to the position of the ribbon.

### BRIEF SUMMARY OF THE INVENTION

The present invention provides a system as defined in claim 1. Preferred embodiments are defined in the dependent claims.

The foregoing and other aspects, features, details, utilities, and advantages of the present invention will be apparent from reading the following description and claims, and from reviewing the accompanying drawings.

### BRIEF DESCRIPTION OF THE DRAWINGS

FIG. 1 generally illustrates a diagrammatic view of a system including an embodiment of a catheter connected to an electronic control unit (ECU) and a display.
FIG. 2 generally illustrates a side view of the embodiment of the catheter having an ultrasound transducer assembly, body, handle, electrical connector, and wire harness of FIG 1.
FIG. 3 is an illustration of an embodiment of an ultrasound transducer assembly in various configurations with a plurality of wire harnesses.
FIG. 4 is an illustration of a cross-sectional side view of an embodiment of the handle of FIG. 1
FIG. 5 is an illustration of an embodiment of a wire harness.
FIG. 6 is a general illustration of an embodiment of a plurality of twisted pair wiring.
FIG. 7 is an illustration of an embodiment of an electrical connector having a plurality of zero insertion force (ZIF) connectors configured to receive a plurality of wire harnesses.
FIG. 8 is an illustration of the ZIF connector of FIG. 7, configured to receive a proximal end of the wire harness of FIG. 5.
FIG. 9 is a general illustration of an embodiment of a distal flex circuit and a proximal flex circuit of the wire harness of FIG. 5.
FIG. 10 is a general illustration of another embodiment of a distal flex circuit and a proximal flex circuit of the wire harness of FIG. 5.

### DETAILED DESCRIPTION OF THE INVENTION

Various embodiments are described herein to various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments. The scope of the invention is defined by the appended claims.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting or absolute.

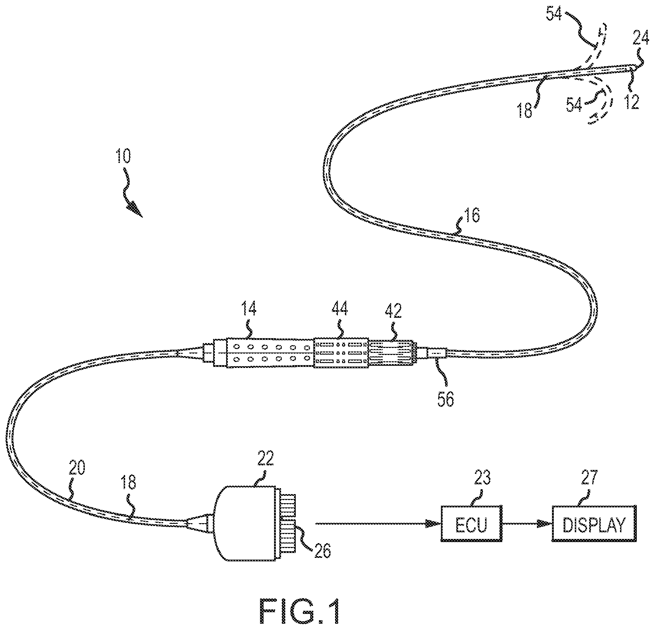

Referring now to the drawings wherein like reference numerals are used to identify identical components in the various views, FIGS. 1-2 generally illustrates an exemplary embodiment of a catheter 10 for performing one or more diagnostic and/or therapeutic functions. More particularly, the catheter 10 may include components for performing intra-cardiac echocardiography ("ICE") procedures. It should be understood, however, that while the description below is with respect to an ICE catheter, the subject matter of the disclosure may find application in connection with a variety of medical devices. As generally illustrated in FIG. 1, a catheter 10 may comprise an ultrasound transducer assembly 12, a handle 14, a body 16, a wire harness 18, a tubing 20, and an electrical connector 22 configured to connect to an electronic control unit (ECU) 23, such as, for example and without limitation, the ViewMate™ Z or ViewMate™ II intracardiac ultrasound consoles via the compatible ViewFlex™ Catheter Interface Module commercialized by St. Jude Medical, Inc. In an embodiment, the ultrasound console may have a system frequency of 4.5 - 8.5 MHz. In another embodiment, the system frequency may be 3.0 - 9.0 MHz. In an embodiment, the ultrasound console may have a viewing angle of 90° F. In another embodiment, the viewing angle may be 80° F. In an embodiment, the ultrasound consoles may have a maximum viewing depth of 18 cm. The catheter 10 may have a distal end 24 and a proximal end 26. In an embodiment, the ultrasound imaging modes may comprise a B-mode, M-mode, Spectral (Pulse Wave Doppler) Doppler, and/or Color Doppler.

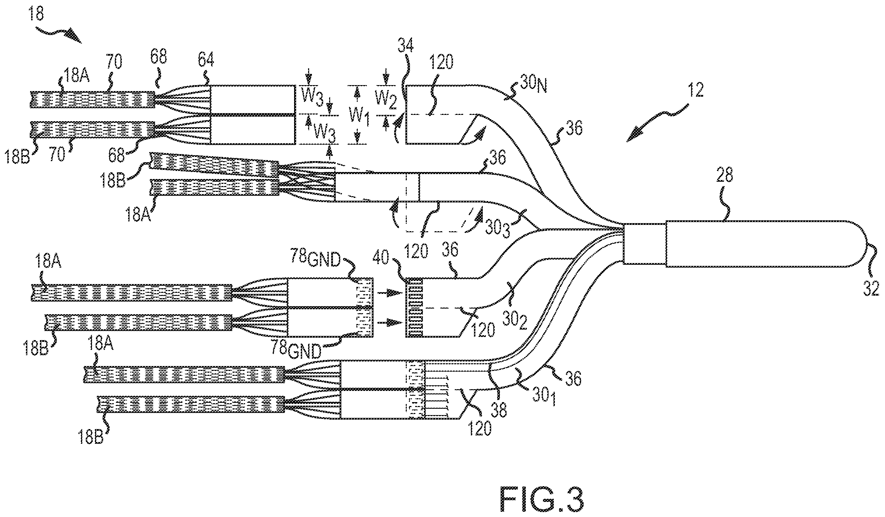

Referring to FIG. 3, an ultrasound transducer assembly 12 may comprise a transducer 28 and a plurality of flexible printed circuits 30. The ultrasound transducer assembly may have a distal end 32 and a proximal end 34. The transducer 28 may include a plurality of piezoelectric elements that are operatively or conductively connected to the flexible printed circuits 30. Each flexible printed circuit 30 may comprise a flexible substrate 36, a plurality of conductive traces 38 defined on the substrate 36, and a plurality of conductive pads 40. Each piezoelectric element may be operatively or conductively connected to a separate trace 38 defined on the substrate 36 of the flexible printed circuit 30. In an exemplary embodiment, the piezoelectric elements of the transducer may be operatively or conductively connected to the plurality of traces 38 by trace-to-trace soldering using techniques such as reflow (hot bar) soldering or anisotropic conductive film (ACF) bonding which use a combination of pressure, heat, and time. In an embodiment, the transducer 28 may, without limitation, comprise a sixty-four (64) element linear phased array comprised of piezoelectric composite (PZT) materials. In an embodiment, the transducer 28 may be housed in silicone. In an embodiment, the transducer 28 may be visible with or under fluoroscopy.

In an exemplary embodiment, the ultrasound transducer assembly 12 may have a transducer 28 having sixty-four (64) piezoelectric elements and four flexible printed circuits 30 operatively or conductively connected to the transducer 28. Each of the four flexible printed circuits 30 may, for example, include eighteen (18) traces 38 and eighteen (18) pads 40, wherein each individual trace 38_{N} may be operatively or conductively connected to each individual pad 40_{N}, creating a trace/pad circuit. In such an embodiment, eighteen (18) conductive traces 38₁₋₁₈ and eighteen (18) conductive pads 40₁₋₁₈ may form eighteen (18) trace/pad circuits, wherein two (2) trace/pad circuits may be connected to ground wires, and the remaining sixteen (16) trace/pad circuits may be connected to sixteen (16) piezoelectric elements of the transducer 28. In an exemplary embodiment, the two outermost trace/pad circuits may correspond to ground wiring, and the sixteen (16) inner trace/pad circuits may correspond to 16 separate piezoelectric elements. For example, and without limitation, piezoelectric elements one (1) through sixteen (16) may correspond with a first flexible printed circuit 30₁, piezoelectric elements seventeen (17) through thirty-two (32) may correspond with a second flexible printed circuit 30₂, piezoelectric elements thirty-three (33) through forty-eight (48) may correspond with a third flexible printed circuit 30₃, and piezoelectric elements forty-nine (49) through sixty-four (64) may correspond with a fourth flexible printed circuit 304. While the ultrasound transducer assembly 12 has been described with four flexible printed circuits 30₁₋₄, the present disclosure is not meant to be so limited. Rather, a plurality of flexible printed circuits 30_{N} may be used as required and remain with the spirit and scope of the present disclosure.

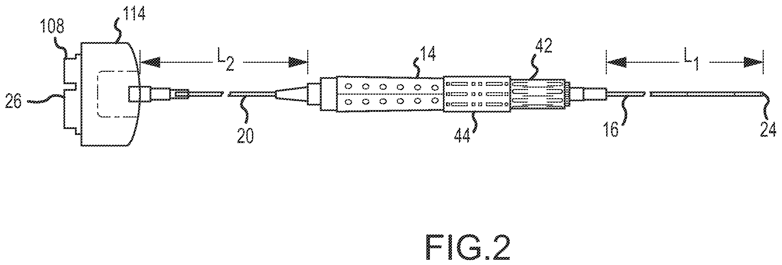

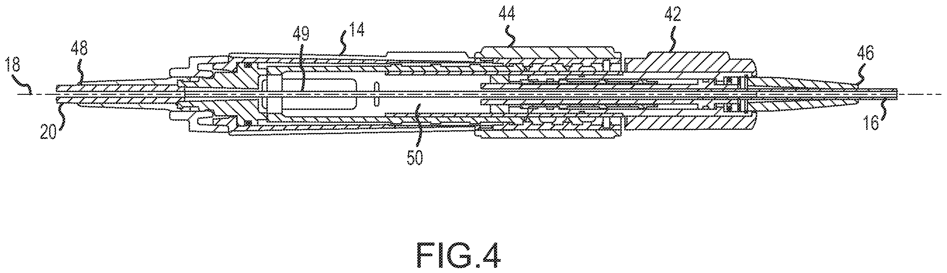

As generally illustrated in FIGS. 1, 2, and 4, an embodiment of the handle 14 may be similar to a handle described in United States patent application publication no. 2011/0282176 A1 published on 17 November 2011. In embodiments, the handle 14 may provide for four-way steering of the distal end portion of the body 16 of the catheter 10 (described in further detail below) and/or the ultrasound transducer assembly 12 by manipulation of two or more control members. As seen in FIG. 2, embodiments of the handle 14 may include an actuator or knob 42 or a plurality of actuators or knobs, such as a first knob 42 and a second knob 44, which may be configured to manipulate two or more control members. As seen in FIG. 4, the handle 14 may have a distal end 46 and a proximal end 48. The wire harness 18, located in a lumen of the body 16, may enter the distal end 46 of the handle 14, pass internally through the handle 14 via a passage 50, and exit the proximal end 48 of the handle 14. In an embodiment, a portion of the wire harness 18 may be surrounded by a protective sleeve 49 in the passage 50 of the handle 14. After the wire harness 18 passes through the handle 14, the wire harness 18 may pass into a lumen of the tubing 20. In an embodiment, the wire harness 18 may directly pass through the passage 50 in the handle 14 along a path substantially parallel to a central longitudinal axis 52 of the handle 14. In another embodiment, a portion of the wire harness 18 may be provided off-center of the handle 14 when the wire harness 18 exits the proximal end of the handle 14.

As generally illustrated in FIGS. 1 and 2, the body 16 of the catheter 10 may comprise a flexible tubular body 16 having a deflectable distal end portion 54 and a proximal end 56. The deflection of the distal end portion 54 of the body 16 may be actuated by the handle 14 and the deflection of distal end portion 54 may be multi-directional. In an embodiment, the distal end portion 54 of the body 16 may be configured for four-directional deflection allowing for left-right and posterior-anterior deflection, with an angle of at least about 120 degrees in each direction. In an embodiment, the distal end portion 54, which may be deflectable, may be approximately 11.4 cm (four and one-half (4-1/2) inches) in length. In another embodiment, the deflectable length may be approximately 11.68 cm ± 0.50 cm. In an embodiment, the distal end portion 54 of the body 16 may be deflected in a posterior-anterior direction, for example, by rotating the first knob 42 located on the handle 14. In an embodiment, the anterior/posterior deflection may be separated from each other by 180 degrees nominal and ± 25 degrees from a plane described by a neutral indicator on the handle 14 and a center axis of the distal end portion 54 of the body 16. In an embodiment, the distal end portion 54 of the body 16 may also be deflected in a left-right deflection by rotating the second knob 44 located on the handle 14. In an embodiment, the left-right deflection may be separated from each other by 180 degrees nominal and ± 25 degrees from the plane described by the neutral indicator on the handle 14 and a center axis of the distal end portion 54 of the body 16. In another embodiment, the first knob 42 may control the left-right deflection and second knob 44 may control the posterior-anterior direction. In an embodiment, the body 16 may be cylindrical and electrically non-conductive. In an embodiment, the body 16 may have a 9 French (F) diameter. In an embodiment where the body 16 has a 9F diameter, a 10F introducer may for example be used with the 9F body for insertion into the femoral or jugular veins. In an embodiment, the body 16 may comprise a radio-opaque Pebax™ tubing. In an embodiment, the body 16 may have a usable length ("L1") of about 90 cm. In an embodiment, the ultrasound transducer assembly 12 may be located at the distal end portion 54 of the body 16 and the wire harness 18 (such as for example described in further detail below) attached to the ultrasound transducer assembly 12 may extend through the body 16 from substantially the distal end 24 to the proximal end 26 of the catheter 10. In an embodiment, the body 16, and the wire harness 18 extending through the body 16, may enter an opening of the handle 14.

As previously described, the wire harness 18 may pass completely through the handle 14, such that the handle 14 is between a distal end 58 of the wire harness 18 and a proximal end 60 of the wire harness 18. In an embodiment, the portion of the wire harness 18 between the handle 14 and proximal end 60 of the wire harness 18 may be covered by the tubing 20. In an embodiment, the tubing 20 may be connected to the connector 22 and the handle 14, and the tubing 20 may protect and package the wire harness 18. In an embodiment, the tubing 20 and the wire harness 18 extending through the tubing 20, such as shown in FIG. 2, may have a length ("L2") that may be about 110 cm long.

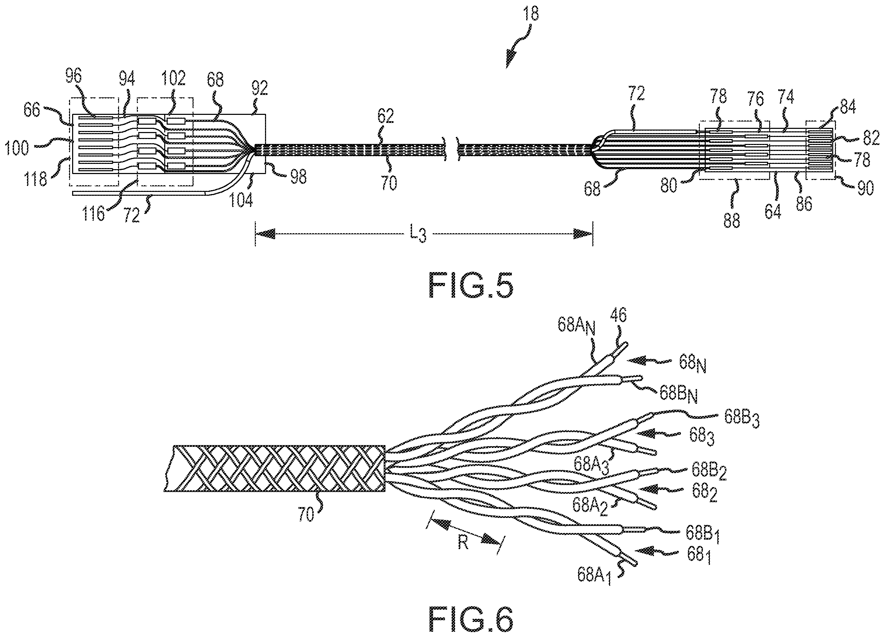

Referring to FIGS. 5-10, the wire harness 18 comprises a wiring bundle 62, a distal flex circuit 64, and a proximal flex circuit 66. The wire harness 18 may be configured to electrically connect an ultrasound transducer assembly 12 to the electrical control unit (ECU) 23, wherein the ECU 23 may control various functions of the transducer 28, including but not limited to, capturing and recording signal data obtained from the transducer 28, and providing images based on transducer 28 data via a display device 27 connected to the ECU 23.

The wiring bundle 62 may have a distal portion and a proximal portion. The wiring bundle 62 of the wire harness 18 comprises a plurality of twisted pair wiring 68 (or twisted wire pairs) and a shield 70, as shown in FIGS. 5 and 6. As generally illustrated in FIG. 6, each twisted pair wiring may comprise two wires 68A, 68B with insulation surrounding each individual wire 68A, 68B. The two individual wires 68A, 68B may then be twisted around each other, forming the twisted pair wiring 68. The rate or frequency of twisting ("R") may, for example and without limitation, be 27 turns per 2.54 cm (1 inch). In an exemplary embodiment, the wires 68A, 68B may be forty-six (46) gauge wiring and each individual wire 68A, 68B may be color coded, wherein the color may be predefined to connect to a particular circuit. In an exemplary embodiment, the plurality of twisted pair wiring 68 may be bound together in a straight lay routing orientation, where each twisted pair wiring 68 is not twisted around another twisted pair wiring 68. A straight lay routing orientation may provide benefits, such as minimizing the cross-sectional diameter of the wiring bundle 62 and/or maximizing the flexibility of the wiring bundle 62. In an exemplary embodiment, the plurality of twisted pair wiring 68 may comprise eight twisted pair wirings 68. The plurality of twisted pair wiring 68 bound together may be secured with the shield 70. In an exemplary embodiment, the shield 70 may be braided and may have a pigtail wire 72 running throughout the length of the shield 70. In an exemplary embodiment, the shield 70 may comprise a silver plated copper braiding. In an exemplary embodiment, the shield 70 may be forty-six (46) gauge. In an exemplary embodiment, the shield 70 may cover at least 90% of the length of the plurality of twisted pair wiring 68. For some embodiments, the shield 70 may have a length ("L3") as much as 254 cm (100 inches) or more. In an exemplary embodiment, one end of the shield 70 may be located approximately 1.3 cm (one-half inch (1/2 inch)) from an edge 80 of the distal flex circuit 64 and the other end of the shield 70 may be located approximately 1.3 cm (one-half inch (1/2 inch)) from an edge 98 of the proximal flex circuit 66.

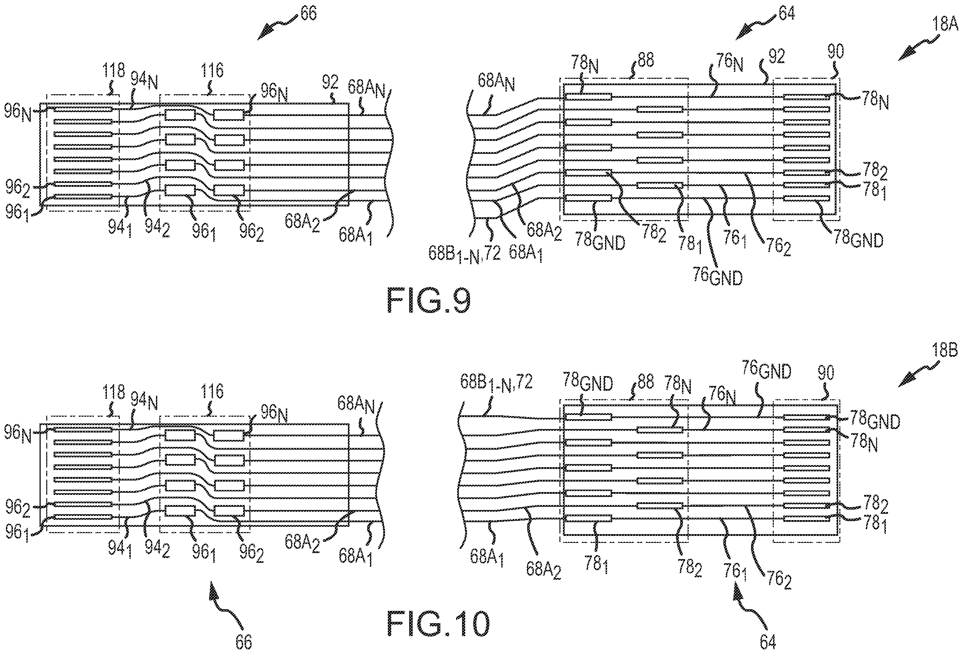

As generally illustrated in FIGS. 5 and 10, the distal flex circuit 64 of the wire harness 18 may be a flexible printed circuit 30 comprising a flexible substrate 74, a plurality of conductive traces 76, and a plurality of conductive pads 78.

The substrate 74 may have a generally thin thickness (cross-sectional area) which may allow the substrate 74 to flex, bend, and fold. The shape of the substrate 74 may be defined by a first edge 80, a second edge 82, a third edge 84, and a fourth edge 86. The first edge 80 (or proximal edge) may be located closest to the end of the shield 70 of the wiring bundle 62. The second edge 82 (or distal edge) may be located opposite the first edge 80. The third and fourth edges 84, 86 may be each adjacent the first and second edges 80, 82, and the third and fourth edges 84, 86 may each connect to the first and second edges 80, 82. In an embodiment, the substrate 74 may be substantially rectangular in shape, where the shape is defined by the first edge 80, second edge 82, third edge 84, and fourth edge 86. In an embodiment, the substrate 74 may comprise a dielectric material, such as, but not limited to, polyimide.

The plurality of conductive traces 76 and the plurality of conductive pads 78 may be defined on the substrate 74. Predetermined traces 76 and pads 78 may be combined, creating trace/pad combinations configured to create electrically conductive circuits on the flexible circuit. The pads 78 may be generally larger in width than the traces 76. In an embodiment, the pads 78 may be configured for receiving a solder joint to connect a particular wire to a particular circuit created trace/pad combination. In another embodiment, the pads 78 may be configured to mate to a corresponding set of pads 78 located on one of the plurality of flexible printed circuits 30 of the ultrasound transducer assembly 12. In an embodiment, the electrically conductive bond between the distal flex circuit 64 of the wire harness 18 and the flexible printed circuit 30 of the ultrasound transducer assembly 12 may be connected using reflow (hot bar) soldering. In another exemplary embodiment, an anisotropic conductive film (ACF) may be used for the electrically conductive bond by placing the ACF between the distal flex circuit 64 of the wire harness 18 and the flexible printed circuit 30 of the ultrasound transducer assembly 12.

In an exemplary embodiment, the distal flex circuit 64 may have two sets of conductive pads 78, a first set 88, and a second set 90. Each set 88, 90 may have a plurality of conductive pads 78 where the number of pads 78 in a set is equal to the number of defined electrical circuits on the distal flex circuit 64. For example, in an exemplary embodiment, the first set 88 may have nine pads 78, and the second set 90 may have nine pads 78. Each pad 78 in the set 88, 90 may be part of a defined circuit connected by the traces 76. In other words, the first pad 78 in the first set 88 may be connected to the first pad 78 in the second set 90, wherein each pad 78 is connected by the traces 76. Thus, the embodiment having nine pads 78 per set 88, 90 would have nine separate circuits. While the description above describes sets 88, 90 having nine pads 78, it will be appreciated that the present disclosure is not meant to be so limited. Rather, other exemplary embodiments may use any number of pads 78 per set to accommodate the desired amount of electrical circuits. Accordingly, it will be appreciated that embodiments other than those described with particularity herein remain within the spirit and scope of the present disclosure.

In an exemplary embodiment, a first set of pads 78 may be attached to the twisted pair wiring 68 of the wire bundle 62 and the pigtail wiring 72 of the shield 70. For example, in an embodiment having a wiring bundle 62 with eight twisted pair wiring 68, each set of pads 78 on the distal flex circuit 64 may have nine pads 78. One wire 68A from each of the eight twisted pair wiring 68 may be soldered, or connected via other bonding techniques, to a corresponding pad 78 of the first set 88. The other wires 68B of the twisted pair wirings 68, not directly connected to the first set 88, may be operatively or conductively connected to the pigtail wiring 72 of the shield 70, and the pigtail wiring 72 may then be operatively or conductively connected to a ninth pad 78 of the first set 88. In an embodiment, the ninth pad 78 may be used for grounding purposes. In an embodiment, after the twisted pair wiring 68 has been connected to either the first set 88 or the pigtail wiring 72, the connection may be covered with a nonconductive material, such as, but not limited to, epoxy, which may protect the connection. The second set of pads 90, located near the second edge 82 of the substrate 74, may be used to operatively or conductively connect to the flexible printed circuit 30 of the ultrasound transducer assembly 12 using the techniques described above, such as, but not limited to, reflow (hot bar) soldering or ACF. While the above embodiment describes a wire harness 18 using eight twisted pair wiring 68 connected to nine circuits defined on the distal flex circuit 64, any number of twisted pair wiring 68 may be utilized to accommodate the required amount of circuits defined on the distal flex circuit 64.

The proximal flex circuit 66 of the wire harness 18 may be a flexible printed circuit 30 comprising a flexible substrate 92, a plurality of conductive traces 94 defined on the substrate, and a plurality of conductive pads 96 as generally illustrated in FIG. 5.

The substrate 92 may have a generally thin thickness (cross-sectional area) which may allow the substrate 92 to flex, bend, and/or fold. The shape of the substrate may be defined by a first edge 98, a second edge 100, a third edge 102, and a fourth edge 104. The first edge 98 (or distal edge) may be located closest to the end of the shield 70 of the wiring bundle 62. The second edge 100 (or proximal edge) may be located opposite the first edge 98. The third and fourth edges 102, 104 may be each adjacent the first and second edges 98, 100, and the third and fourth edges 102, 104 may each connect to the first and second edges 98, 100. In an embodiment, the substrate 92 may be generally rectangular in shape. In an embodiment, the substrate 92 may comprise a dielectric material, such as, but not limited to, polyimide.

The plurality of traces 94 and the plurality of pads 96 may be defined on the substrate 92. Predetermined traces 94 and pads 96 may be combined, creating trace/pad combinations configured to create electrically conductive circuits on the flexible circuit. The pads 96 may have a generally larger width than the traces 94. In an embodiment, the pads 96 may be configured for receiving a solder joint to connect a particular wire 68A to a particular circuit created by the trace/pad combination.

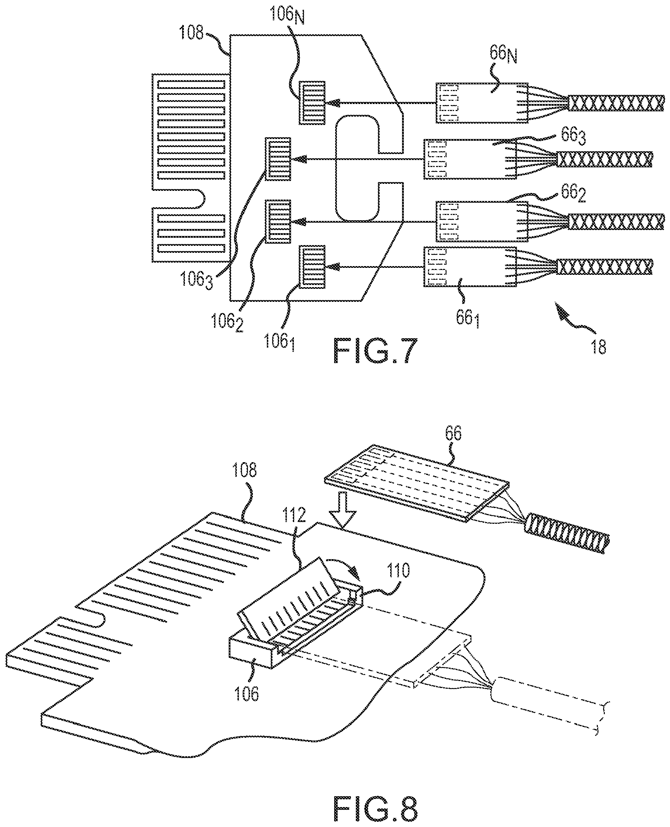

As generally illustrated in FIGS. 7-8, another embodiment of the pads 96 and substrate 92 of the proximal flex circuit 66 may be configured to mate to at least one zero insertion force (ZIF) connector 106 located on a printed circuit board 108, such as a cable edge card, of the electrical connector 22. The ZIF connector 106 may be configured to receive the proximal flex circuit 66. The pads 96 located on the substrate 92 of the proximal flex circuit 66 may be placed directly inside or on a first portion 110 of the ZIF connector 106. A second portion 112 of the ZIF connection 106 may engage the first portion 110 of the ZIF connection 106, causing a conductive connection between the ZIF connector 106 and the proximal flex circuit 66, as well as physically gripping of the substrate 92 of the proximal flex circuit 66. An advantage associated with some ZIF connection systems is that the system does not require a mating half to be fitted to the flexible printed circuit 30, such as the proximal flex circuit 66, which can save space and cost of reduced or miniaturized equipment. In an embodiment, the printed circuit board 108 (such as a rigid cable edge card) having at least one ZIF connector 106 may be configured to electrically connect to the ECU 23. The printed circuit board (such as a rigid cable edge card) may be located in a housing 114 of the electrical connector 22. The electrical connector 22 may connect to the ECU 23.

As seen in FIGS. 5, 9, and 10, embodiments of the proximal flex circuit 66 may have two sets of pads 96, a first set 116 and a second set 118. Each set 116, 118 may have a plurality of pads where the number of pads 96 in a set 116, 118 matches the number of defined electrical circuits on a proximal flex circuit 66. For example, in an embodiment, the first set 116 may have eight pads 96 and the second set 118 may also have eight pads 96. Each pad 96 in the set 116, 118 may be part of a defined circuit connected by the traces 94. In other words, the first pad 96 in the first set 116 may be connected to the first pad 96 in the second set 118, wherein each pad 96 is connected by a trace 94. Thus, an embodiment having eight pads 96 per set 116, 118 may, for instance, have eight separate circuits.

In an embodiment, a first set 116 of pads 96 may be attached to twisted pair wiring 68 of a wire bundle 62. For example, without limitation, in an embodiment having a wiring bundle 62 with eight twisted pair wiring 68, the first set 116 of pads 96 on the proximal flex circuit 66 may have eight pads 96. One wire 68A from each of the eight twisted pair wiring 68 may be soldered, or attached via other electrically connected bonding techniques, to a corresponding pad 96 of the first set 116. The other wires 68B of the twisted pair wirings 68, not directly connected to the first set 116 of pads 96, may be connected to the pigtail wiring 72 of the shield 70, and may then be wrapped and not connected to the proximal flex circuit 66. The pigtail wiring 72 combined with the other wires 68B of the twisted pair wirings 68 located by the proximal end 60 of the wire harness 18 may be secured to the shield 70. In an embodiment, after the twisted pair wiring 68A has been connected to the first set 116 of pads 96, the connection may be covered with a nonconductive material, such as, but not limited to, epoxy, which may protect the connection. The second set 118 may be located near the second edge 100 and the proximal flex circuit 66 with the second set 118 of pads 96 may be inserted into the ZIF connector 106 located on the printed circuit board 108. In an embodiment, the printed circuit board 108 may be connected to, for example, a St. Jude Medical ViewFlex™ Catheter Interface Module which may be connected to a St. Jude Medical ViewMate™ Z or ViewMate™ II intracardiac ultrasound console. While the above embodiment describes a wire harness 18 using eight twisted pair wiring 68 connected to eight circuits defined on the proximal flex circuit 66, any number of twisted pair wiring 68 may be utilized to accommodate the required amount of circuits defined on the proximal flex circuit 66.

The various embodiments of the wire harness 18 described above discloses a singular wire harness 18 electrically connecting the ultrasound transducer assembly 12 to the electrical connector configured to electrically connect to the ECU 23. It will be appreciated, however, that a plurality of wire harnesses 18 may be used to electrically connect the ultrasound transducer assembly 12 to the ECU 23 as seen in FIG. 3. In accordance with the invention, a pair of wire harnesses is provided. There may be various benefits to using a plurality of wire harnesses 18, including without limitation, maximizing flexibility of the medical device using the wire harnesses 18, reduced size of the flex circuits for packaging the wire harness 18 in the catheter 10, and utilizing economies of scale by using common wire harnesses 18 on different medical devices.

For example, and without limitation, in the previously described embodiment of an ultrasound transducer assembly 12 having sixty-four (64) piezoelectric elements and four flexible printed circuits 30 electrically connected to the transducer 28, each of the four flexible printed circuits 30 may have eighteen (18) separate circuits for a total of seventy-two (72) circuits (sixty-four (64) circuits to electrically conduct the signal between the piezoelectric elements and ECU 23 and eight ground circuits). Eight separate wire harnesses 18 may be used to connect the ultrasound transducer assembly 12 to the ECU 23, two wire harnesses 18 per flexible printed circuit 30 of the ultrasound transducer assembly 12.

As seen in FIGS. 9 and 10, embodiments of the flexible printed circuit 30 of the ultrasound transducer assembly 12 may have the ground circuits located on the outermost circuits. These embodiments may allow the first and second wire harness 18A, 18B to match the position of the outermost ground circuits of the ultrasound transducer assembly 12 when connecting the wire harnesses 18A, 18B to the ultrasound transducer assembly 12. The first wire harness 18A and the second wire harnesses 18B may differ only in the location of the ground circuit on the distal flex circuit 64. For example, as seen in FIG. 9, the distal flex circuit may have a plurality of circuits created by the combination of the traces 76_{1-N} and pads 78_{1-N} (e.g., trace/pad circuits 76_{1-N}, 78_{1-N}). The first wire harness 18A may have a ground circuit 76_{GND}, 78_{GND} located adjacent the first trace/pad combination 76₁, 78₁. As seen in FIG. 10, the second wire harness 18B may have the ground circuit 76_{GND}, 78_{GND} located adjacent the N^{th} trace/pad combination 76_{N}, 78_{N}. In both the first and second wire harnesses 18A, 18B, the ground circuit may be one of the outermost trace/pad circuits. In an embodiment, the two wire harnesses 18 may be color coded. For example, the first wire harness 18A may be color coded red and the second wire harness 18B may be color coded black.

As seen in FIG. 3, in an embodiment where two wire harnesses 18 are attached to each flexible printed circuit 30 of the ultrasound transducer assembly 12, each flexible printed circuit 30 of the ultrasound transducer assembly 12 may be folded over onto itself along a fold line 120 oriented generally parallel to at least a portion of the traces 38. By folding the flexible printed circuit 30 of the ultrasound transducer assembly 12, the width ("W₁") of the flexible printed circuit 30 of the ultrasound transducer assembly 12 may be reduced to a smaller width ("W₂"), which may be approximately the same width ("W₃") as the distal flex circuits 64 of the wire harnesses 18. Use of the various embodiments of the wire harnesses 18 disclosed herein, may provide the benefit of improved and/or consistent flexibility of the body 16 when maneuvered in multiple directions by the control handle 14.

Although only certain embodiments have been described above with a certain degree of particularity, those skilled in the art could make numerous alterations to the disclosed embodiments without departing from the scope of the invention as defined in the claims. It is intended that all matter contained in the above description or shown in the accompanying drawings shall be interpreted as illustrative only and not limiting. Changes in detail or structure may be made without departing from the invention as defined in the appended claims.

## Claims

1. A system comprising:
a medical device (10) comprising an elongate body (16) with a lumen, and an electrically controlled object (12);
and a pair of wire harnesses (18) comprising a first wire harness and a second wire harness, each of the first and second wire harnesses comprising:
a wiring bundle (62) comprising:
a distal portion and a proximal portion;
a plurality of twisted pair wiring (68); and
a shield (70) surrounding a portion of the plurality of twisted pair wiring (68);
a distal flex circuit (64) connected to the distal portion of the wiring bundle (62), the distal flex circuit configured to operatively connect to a flexible printed circuit (30) of the electrically controlled object; wherein the first wire harness and the second wire harness are each operatively connected to the same flexible printed circuit (30) of the electrically controlled object, the flexible printed circuit (30) being configured to be folded over onto itself along a fold line (120) to reduce its width; and
a proximal flex circuit (66) connected to the proximal portion of the wiring bundle (62), the proximal flex circuit (66) configured to operatively connect to an electrical connector (22);
wherein each of the first and second wire harnesses (18) is sized and configured to extend substantially through the lumen of the elongate body (16) of the medical device (10).

2. The system of claim 1, wherein the plurality of twisted pair wiring (68) are provided in a straight lay orientation.

3. The system of claim 1 or 2, wherein the electrically controlled object is an ultrasound transducer assembly comprising an ultrasound transducer and a plurality of flexible printed circuits (30), and the distal flex circuit (64) is configured to operatively connect to at least one of the plurality of flexible printed circuits (30).
St. Jude Medical, Atrial Fibrillation Division, Inc.

4. The system of claim 3, wherein the operative or conductive connection between the distal flex circuit (64) and the at least one of the plurality of flexible printed circuits (30) is bonded or soldered.

5. The system of claim 3, wherein the operative connection between the distal flex circuit (64) and the at least one of the plurality of flexible printed circuits (30) is bonded with anisotropic conductive film provided between the distal flex circuit (64) and the at least one of the plurality of flexible printed circuits (30).

6. The system of any one of claims 1 to 5, wherein the proximal flex circuit (64) comprises a flexible substrate (74) and a plurality of conductive pads (78), and the flexible substrate and the plurality of conductive pads (78) are configured to connect to a zero insertion force (ZIF) type connector.

7. The system of claim 6, wherein the ZIF type connector is provided on a rigid printed circuit board.

8. The system of any one of claims 1 to 7, wherein the medical device (10) comprises a handle (14), and wherein the wire harness (18) is configured to extend completely through the handle (14).

9. The system of any one of claims 1 to 8, wherein the wiring bundle (62) comprises at least eight twisted pair wiring (68).

10. The system of any one of claims 1 to 9, wherein the twisted pair wiring (68) has a twist rate of at least 27 turns per 2.54 cm (1 inch).

11. The system of any one of claims 1 to 10, wherein the shield (70) comprises a pigtail wire configured for grounding, the twisted pair wiring (68) comprises a first wire and a second wire, and the proximal flex circuit (66) comprises a flexible substrate (30) and a plurality of conductive pads (96), and wherein the first wire of the twisted pair wiring is operatively terminated on a separate conductive pad of the plurality of conductive pads (96), and the second wire is operatively connected to the pigtail wire.

12. The system of any one of claims 1 to 10, wherein the shield (70) comprises a pigtail wire configured for grounding, the twisted pair wiring (68) comprises a first wire and a second wire, and the distal flex circuit (64) comprises a flexible substrate (30) and a plurality of conductive pads (78), and wherein the first wire of the twisted pair wiring (68) is operatively terminated on a first conductive pad of the plurality of conductive pads (78), the second wire is operatively connected to the pigtail wire, and wherein the pigtail wire (72) is operatively terminated on a second conductive pad of the plurality of conductive pads (78).

13. The system of claim 12, wherein the electrically controlled object comprises at least one flexible printed circuit (30) comprising a set of conductive pads, and wherein the distal flex circuit (64) has a set of conductive pads (78) provided proximate a distal end of the distal flex circuit (64), wherein the set of conductive pads (78) have not been directly connected to a first wire of the twisted pair wiring (68), and wherein the set of conductive pads (78) of the distal flex circuit (64) are configured to conductively bond to the set of conductive pads (78) of the electrically controlled object.

## Patentansprüche

1. System mit:
einer medizinischen Vorrichtung (10), die einen länglichen Körper (16) mit einem Lumen und ein elektrisch gesteuertes Objekt (12) aufweist;
und einem Paar von Kabelsträngen (18), die einen ersten Kabelstrang und einen zweiten Kabelstrang aufweisen, wobei jeder von dem ersten und zweiten Kabelstrang aufweist:
ein Kabelbündel (62) mit:
einem distalen Bereich und einem proximalen Bereich;
einer Mehrzahl von verdrillten Paardrähten (68); und
einer Abschirmung (70), die einen Bereich der Mehrzahl von verdrillten Paardrähten (68) umgibt;
eine distale flexible Schaltung (64), die mit dem distalen Bereich des Kabelbündels (62) verbunden ist, wobei die distale flexible Schaltung konfiguriert ist zur operativen Verbindung mit einer flexiblen gedruckten Schaltung (30) des elektrisch gesteuerten Objekts; wobei der erste Kabelstrang und der zweite Kabelstrang jeweils operativ verbunden sind mit der gleichen flexiblen gedruckten Schaltung (30) des elektrisch gesteuerten Objekts, wobei die flexible gedruckte Schaltung (30) konfiguriert ist, um auf sich selbst entlang einer Faltlinie (120) gefaltet zu werden, um deren Breite zu reduzieren; und
eine proximale flexible Schaltung (66), die mit dem proximalen Bereich des Kabelbündels (62) verbunden ist, wobei die proximale flexible Schaltung (66) konfiguriert ist zur operativen Verbindung mit einem elektrischen Verbinder (22);
wobei jeder von dem ersten und zweiten Kabelstrang (18) größenmäßig ausgebildet und konfiguriert ist, um sich im Wesentlichen durch das Lumen des länglichen Körpers (16) der medizinischen Vorrichtung (10) zu erstrecken.

2. System nach Anspruch 1, bei dem die Mehrzahl der verdrillten Paardrähte (68) in einer geraden Lageorientierung bereitgestellt ist.

3. System nach Anspruch 1 oder 2, bei dem das elektrisch gesteuerte Objekt eine Ultraschalltransduceranordnung ist, die einen Ultraschalltransducer und eine Mehrzahl von flexiblen gedruckten Schaltungen (30) aufweist, und die distale flexible Schaltung (64) konfiguriert ist zur funktionellen Verbindung mit mindestens einer von der Mehrzahl von flexiblen gedruckten Schaltungen (30).

4. System nach Anspruch 3, bei dem die funktionelle oder leitende Verbindung zwischen der distalen flexiblen Schaltung (64) und der mindestens einen von der Mehrzahl von flexiblen gedruckten Schaltungen (30) gebonded oder verlötet ist.

5. System nach Anspruch 3, bei dem die operative Verbindung zwischen der distalen flexiblen Schaltung (64) und der mindestens einen von der Mehrzahl von flexiblen gedruckten Schaltungen (30) mit einem anisotropen leitenden Film gebonded ist, die zwischen der distalen flexiblen Schaltung (64) und der mindestens einen von der Mehrzahl von flexiblen gedruckten Schaltungen (30) bereitgestellt ist.

6. System nach einem der Ansprüche 1 bis 5, bei dem die proximale flexible Schaltung (64) ein flexibles Substrat (74) und eine Mehrzahl von leitfähigen Anschlüssen (78) aufweist, und das flexible Substrat und die Mehrzahl der leitenden Anschlüsse (78) konfiguriert sind zur Verbindung mit einem Verbinder vom ZIF (Zero Insertion Force)-Typ.

7. System nach Anspruch 6, bei dem der Verbinder vom ZIF-Typ auf einer starren gedruckten Leiterplatte vorgesehen ist.

8. System nach einem der Ansprüche 1 bis 7, bei dem die medizinische Vorrichtung (10) einen Griff (14) aufweist, und der Kabelstrang (18) konfiguriert ist, um sich vollständig durch den Griff (14) zu erstrecken.

9. System nach einem der Ansprüche 1 bis 8, bei dem das Kabelbündel (62) mindestens acht verdrillte Paarverdrahtungen (68) aufweist.

10. System nach einem der Ansprüche 1 bis 9, bei dem die verdrillte Paarverdrahtung (68) eine Verdrillrate von mindestens 27 Umdrehungen pro 2,54 cm (1 Inch) aufweist.

11. System nach einem der Ansprüche 1 bis 10, bei dem die Abschirmung (70) einen Anschlussdraht aufweist, der konfiguriert ist zur Erdung, wobei die verdrillte Paarverdrahtung (68) einen ersten Draht und einen zweiten Draht aufweist, und die proximale flexible Schaltung (66) ein flexibles Substrat (30) und eine Mehrzahl von leitenden Anschlüssen (96) aufweist, und wobei der erste Draht der verdrillten Paardrahtung funktionell auf einem separaten leitfähigen Anschluss der Mehrzahl von leitenden Anschlüssen (96) terminiert ist, und der zweite Draht funktionell mit dem Anschlussdraht verbunden ist.

12. System nach einem der Ansprüche 1 bis 10, bei dem die Abschirmung (70) einen Anschlussdraht aufweist, der konfiguriert ist zur Erdung, wobei die verdrillte Paarverdrahtung (68) einen ersten Draht und einen zweiten Draht aufweist, und die distale flexible Schaltung (64) ein flexibles Substrat (30) und eine Mehrzahl von leitfähigen Anschlüssen (78) aufweist, und wobei der erste Draht der verdrillten Paarverdrahtung (68) funktionell auf einen ersten leitfähigen Anschluss von der Mehrzahl von leitfähigen Anschlüssen (78) terminiert ist, der zweite Draht funktionell mit dem Anschlussdraht verbunden ist, und der Anschlussdraht (72) funktionell auf einen zweiten leitfähigen Anschluss von der Mehrzahl von leitfähigen Anschlüssen (78) terminiert ist.

13. System nach Anspruch 12, bei dem das elektrisch gesteuerte Objekt mindestens eine flexible gedruckte Schaltung (30) aufweist, die einen Satz von leitfähigen Anschlüssen aufweist, und die distale flexible Schaltung (64) einen Satz von leitfähigen Anschlüssen (78) hat, die proximal zu einem distalen Ende der distalen flexiblen Schaltung vorgesehen sind, wobei der Satz von leitfähigen Anschlüssen (78) nicht direkt mit dem ersten Draht der verdrillten Paardrahtung (68) verbunden ist, und wobei der Satz von leitfähigen Anschlüssen (78) der distalen flexiblen Schaltung (64) konfiguriert ist zur leitenden Verbindung mit dem Satz von leitfähigen Anschlüssen (78) des elektrisch gesteuerten Objekts.

## Revendications

1. Système comprenant :
un dispositif médical (10) comprenant un corps allongé (16) avec une lumière, et un objet commandé électriquement (12) ;
et une paire de faisceaux de câbles (18) comprenant un premier faisceau de câbles et un second faisceau de câbles, chacun des premier et second faisceaux de câbles comprenant :
un ensemble de câblage (62) comprenant :
une partie distale et une partie proximale ;
une pluralité de câbles à paires torsadées (68) ; et
un blindage (70) entourant une partie de la pluralité de câbles à paires torsadées (68) ;
un circuit flexible distal (64) connecté à la partie distale de l'ensemble de câblage (62), le circuit flexible distal étant configuré pour se connecter fonctionnellement à un circuit imprimé flexible (30) de l'objet commandé électriquement ; dans lequel le premier faisceau de câbles et le second faisceau de câbles sont chacun connectés fonctionnellement au même circuit imprimé flexible (30) de l'objet commandé électriquement, le circuit imprimé flexible (30) étant configuré pour être replié sur lui-même suivant une ligne de pli (120) pour réduire sa largeur, et
un circuit flexible proximal (66) connecté à la partie proximale de l'ensemble de câblage (62), le circuit flexible proximal (66) étant configuré pour se connecter de manière opérationnelle à un connecteur électrique (22) ;
dans lequel chacun des premier et second faisceaux de câbles (18) est dimensionné et configuré pour s'étendre sensiblement à travers la lumière du corps allongé (16) du dispositif médical (10).

2. Système selon la revendication 1, dans lequel la pluralité de câbles à paires torsadées (68) sont prévus dans une orientation droite.

3. Système selon la revendication 1 ou 2, dans lequel l'objet commandé électriquement est un ensemble transducteur à ultrasons comprenant un transducteur à ultrasons et une pluralité de circuits imprimés flexibles (30), et le circuit flexible distal (64) est configuré pour se connecter de manière opérationnelle à au moins un circuit parmi la pluralité de circuit imprimé flexible (30).

4. Système selon la revendication 3, dans lequel la connexion fonctionnelle ou conductrice entre le circuit flexible distal (64) et ledit au moins un circuit parmi la pluralité de circuits imprimés flexibles (30) est liée ou soudée.

5. Système selon la revendication 3, dans lequel la connexion fonctionnelle entre le circuit flexible distal (64) et ledit au moins un circuit parmi la pluralité de circuits imprimés flexibles (30) est liée par un film conducteur anisotrope prévu entre le circuit flexible distal (64) et ledit au moins un circuit parmi la pluralité de circuits imprimés flexibles (30).

6. Système selon l'une quelconque des revendications 1 à 5, dans lequel le circuit flexible proximal (64) comprend un substrat flexible (74) et une pluralité de pads conducteurs (78), et le substrat flexible et la pluralité de pads conducteurs (78) sont configurés pour se connecter à un connecteur de type force d'insertion nulle (ZIF).

7. Système selon la revendication 6, dans lequel le connecteur de type ZIF est prévu sur un circuit imprimé rigide.

8. Système selon l'une quelconque des revendications 1 à 7, dans lequel le dispositif médical (10) comprend une poignée (14), et dans lequel le faisceau de câbles (18) est configuré pour s'étendre complètement à travers la poignée (14).

9. Système selon l'une quelconque des revendications 1 à 8, dans lequel l'ensemble de câblage (62) comprend au moins huit paires torsadées (68).

10. Système selon l'une quelconque des revendications 1 à 9, dans lequel le câblage à paires torsadées (68) a une vitesse de torsion d'au moins 27 tours par 2,54 cm (1 pouce).

11. Système selon l'une quelconque des revendications 1 à 10, dans lequel le blindage (70) comprend un câble queue de cochon configuré pour la mise à la terre, le câblage à paire torsadée (68) comprend un premier câble et un second câble, et le circuit flexible proximal (66) comprend un substrat flexible (30) et une pluralité de pads conducteurs (96), et dans lequel le premier câble du câble de paire torsadée est terminé de manière fonctionnelle sur un pad conducteur distinct de la pluralité de pads conducteurs (96) et le second câble est connecté en fonctionnement au câble queue de cochon.

12. Système selon l'une quelconque des revendications 1 à 10, dans lequel le blindage (70) comprend un câble queue de cochon configuré pour la mise à la terre, le câblage à paire torsadée (68) comprend un premier câble et un second câble, et le circuit flexible distal (64) comprend un substrat flexible (30) et une pluralité de pads conducteurs (78), et dans lequel le premier câble du câblage à paires torsadées (68) est terminé de manière fonctionnelle sur un premier pad conducteur de la pluralité de pads conducteurs (78), le second câble est connecté de manière fonctionnelle au câble queue de cochon, et dans lequel le câble queue de cochon (72) est terminé de manière fonctionnelle sur un second pad conducteur de la pluralité de pads conducteurs (78).

13. Système selon la revendication 12, dans lequel l'objet commandé électriquement comprend au moins un circuit imprimé flexible (30) comprenant un ensemble de pads conducteurs, et dans lequel le circuit flexible distal (64) a un ensemble de pads conducteurs (78) prévues à proximité d'une extrémité distale du circuit flexible distal (64), dans lequel l'ensemble de pads conducteurs (78) n'a pas été directement connecté à un premier câble du câblage à paire torsadée (68) et dans lequel l'ensemble des pads conducteurs (78) du circuit flexible distal (64) est configuré pour se lier conductivement à l'ensemble de pads conducteurs (78) de l'objet commandé électriquement.
